# EUROPÄISCHE PATENTSCHRIFT

(11) **EP 1 894 537 B1**
(45) Veröffentlichungstag und Bekanntmachung des Hinweises auf die Patenterteilung: **28.07.2010**
(21) Anmeldenummer: 07115220.1
(22) Anmeldetag: 29.08.2007
(51) Int. Cl.: A61B 18/14, A61B 17/00, A61B 18/00

(54) **HF-Chirurgiegerät und Adapter für dieses Gerät**
HF surgery device and adapter for the device
Appareil de chirurgie HF et adaptateur pour cet appareil

(30) Priorität: 30.08.2006 DE 102006040502
(43) Veröffentlichungstag der Anmeldung: 05.03.2008
(73) Patentinhaber: Aesculap AG, 78532 Tuttlingen (DE)
(72) Erfinder: Keller, Anton, 78589 Dürbheim (DE)
(74) Vertreter: Hoeger, Stellrecht & Partner Patentanwälte

(56) Entgegenhaltungen:
- WO-A-01/37745
- WO-A-2004/045436
- WO-A-2005/043324
- US-A- 4 071 028
- US-A1- 2001 029 315

## Beschreibung

Die Erfindung betrifft ein HF-Chirurgiegerät mit einem HF-Generator, der einen HF-Ausgang und einen Schaltanschluss für ein externes Schaltelement aufweist, und mit mindestens einem bipolaren HF-Instrument, dem von dem HF-Ausgang des HF-Generators elektrische HF-Energie zugeführt wird und das einen Handschalter aufweist.

Für bipolare HF-Instrumente sind HF-Generatoren bekannt, die einen HF-Ausgang aufweisen, an den die HF-Instrumente angeschlossen werden. Über den HF-Ausgang wird Hochfrequenzenergie von dem HF-Generator auf das HF-Instrument übertragen.

Aktiviert, d.h. ein- und ausgeschaltet, werden diese HF-Generatoren üblicherweise durch einen Fußschalter, der als externes Schaltelement mit einem Schaltanschluss des HF-Generators verbunden ist.

Mit derartigen HF-Generatoren kann also ein einziges HF-Instrument versorgt werden, das Ein- und Ausschalten erfolgt über den externen Fußschalter. Wenn ein weiteres HF-Instrument eingesetzt werden soll, muss das zuerst benutzte HF-Instrument von dem HF-Ausgang des HF-Generators gelöst werden und dann muss das weitere HF-Instrument angeschlossen werden, dies bedeutet im Operationssaal, das ein nicht steriler Helfer benötigt wird, um diesen Anschlusswechsel vorzunehmen.

Es sind bipolare Instrumente bekannt, die an den Instrumenten Handschalter tragen, über die die Aktivierung und Deaktivierung des HF-Generators erfolgen kann. Zur Verarbeitung der Signale dieser Handschalter müssen jedoch die HF-Generatoren entsprechend ausgebildet sein, d.h. die HF-Ausgänge müssen zusätzliche Verbindungen für die Signale des Handschalters aufweisen, und dies ist bei vielen HF-Generatoren nicht der Fall, so dass die Handschalter nicht eingesetzt werden können, bei den meisten HF-Generatoren erfolgt die Aktivierung ausschließlich über externe Fußschalter.

Ein Adapter zur Umschaltung zwischen mehreren Instrumenten ist aus WO 01/37745 bekannt, wobei das Schaltsignal nicht zum HF-generator weitergeleitet wird, sondern die Umschaltung im Adapter erfolgt. Ein Adapter mit Weiterleitung eines Schaltsignals von einem Handschalten eines Instruments ist aus US 2001/0029315 bekannt, wobei eine direkte Weiterleitung ohne Umwandlung des Schaltsignals erfolgt.

Es ist Aufgabe der Erfindung, ein gattungsgemäßes HF-Chirurgiegerät so auszubilden, dass auch mit HF-Generatoren, die nur einen HF-Ausgang und einen Schaltanschluss für ein externes Schaltelement aufweisen, der Betrieb von HF-Instrumenten möglich wird, die Handschalter tragen, wobei die Handschalter funktionsgleich den Fußschaltern einsetzbar sein sollen, welche bei einem Betrieb des HF-Generators z ohne Adapter Verwendung finden.

Diese Aufgabe wird erfindungsgemäß durch ein HF-Chirurgiegerät der eingangs beschriebenen Art gelöst, mit einem Adapter mit mindestens einem Anschluss für das HF-Instrument, einem HF-Eingang, der mit dem HF-Ausgang des HF-Generators verbunden ist, mit einem Schaltausgang, der mit dem Schaltanschluss am HF-Generator verbunden ist, und mit elektrischen Schaltelementen, die den Anschluss für das HF-Instrument einerseits mit dem HF-Eingang und andererseits mit dem Schaltausgang verbinden, wobei die Schaltelemente die Signale des Handschalters umwandeln in Schaltsignale für den Schaltanschluss des HF-Generators. So können beispielsweise Impedanzwandler eingesetzt werden oder andere elektrische Schaltelemente, die die Signale des Handschalters so verarbeiten, dass am Schaltausgang des Adapters Signale erzeugt werden, die für den Schaltanschluss des HF-Generators wirksam sind.

Gemäß einer weiteren vorteilhaften Ausgestaltung der Erfindung kann vorgesehen sein, dass die HF-Instrumente Identifikationselemente tragen, die über den Anschluss für ein HF-Instrument an eine Erkennungsschaltung im Adapter angeschlossen sind. Derartige Identifikationselemente können beispielsweise Widerstände unterschiedlicher Größe sein oder aber Transponder mit entsprechenden Daten, die von dem Adapter ausgelesen werden können. Die Datenübertragung erfolgt dabei in jedem Falle über den Anschluss für das HF-Instrument am Adapter.

Vorteilhaft ist es, wenn die Erkennungsschaltung elektrische Sollwerte für das angeschlossene HF-Instrument und elektrische Ist-Werte, die der jeweiligen Einstellung des HF-Generators entsprechen, vergleicht und bei einer Abweichung, die einen vorgegebenen Wert übersteigt, ein Warn- und/oder Abschaltsignal erzeugt.

Der Adapter kann einen Schaltanschluss für ein externes Schaltelement aufweisen, der mit dem Schaltausgang des Adapters in Verbindung steht. An diesem Schaltanschluss kann beispielsweise ein Fußschalter angeschlossen werden, über den ebenfalls die Aktivierung des HF-Generators erfolgt. Dieser Fußschalter kann alternativ oder zusätzlich zu den Handschaltern Verwendung finden, das Signal des externen Schaltelementes wird im Adapter so bearbeitet, dass es am Schaltausgang des Adapters in einer Form vorliegt, die für den Schaltanschluss des HF-Generators verträglich ist.

Vorzugsweise ist der Adapter in einem eigenen Gehäuse angeordnet.

Der Adapter kann mittels Anschlusskabelkabel mit dem HF-Ausgang und dem Schaltanschluss des HF-Generators verbunden sein.

Die Erfindung bezieht sich nicht nur auf ein HF-Chirurgiegerät, welches den HF-Generator, den Adapter und angeschlossene HF-Instrumente betrifft, sondern auch auf den entsprechend ausgebildeten Adapter an sich.

Die nachfolgende Beschreibung bevorzugter Ausführungsformen der Erfindung dient im Zusammenhang mit der Zeichnung der näheren Erläuterung. Es zeigen:
- Figur 1:: ein HF-Chirurgiegerät mit einem HF-Generator, einem Adapter und zwei daran angeschlossenen HF-Instrumenten und
- Figur 2:: eine schematische Draufsicht auf ein HF- Chirurgiegerät gemäß Figur 1 mit nur einem angeschlossenen HF-Instrument.

Das in der Zeichnung dargestellte HF-Chirurgiegerät 1 umfasst mehrere Komponenten, nämlich einen in einem eigenen Gehäuse angeordneten HF-Generator 2, einen ebenfalls in einem eigenen Gehäuse angeordneten Adapter 3 sowie zwei bipolare HF-Instrumente 4 und 5, die im dargestellten Ausführungsbeispiel als Klemme bzw. Pinzette ausgebildet sind.

Das HF-Chirurgiegerät weist einen HF-Ausgang 6 auf, über den HF-Energie abgegeben wird, und einen Schaltanschluss 7, über den dem HF-Generator 2 Signale zum Ein- und Ausschalten zugeführt werden können. Dieser Schaltanschluss 7 kann so ausgebildet sein, dass er normalerweise, d.h. ohne Betrieb des Adapters 3, mit einem Fußschalter in Verbindung steht, der vom Operateur betätigt wird.

Bei Verwendung des Adapters 3 jedoch ist der Schaltanschluss 7 über ein Schaltkabel 8 verbunden mit einem Schaltausgang 9 am Adapter 3. Der HF-Ausgang 6 ist über ein HF-Kabel 10 verbunden mit einem HF-Eingang 11 am Adapter 3.

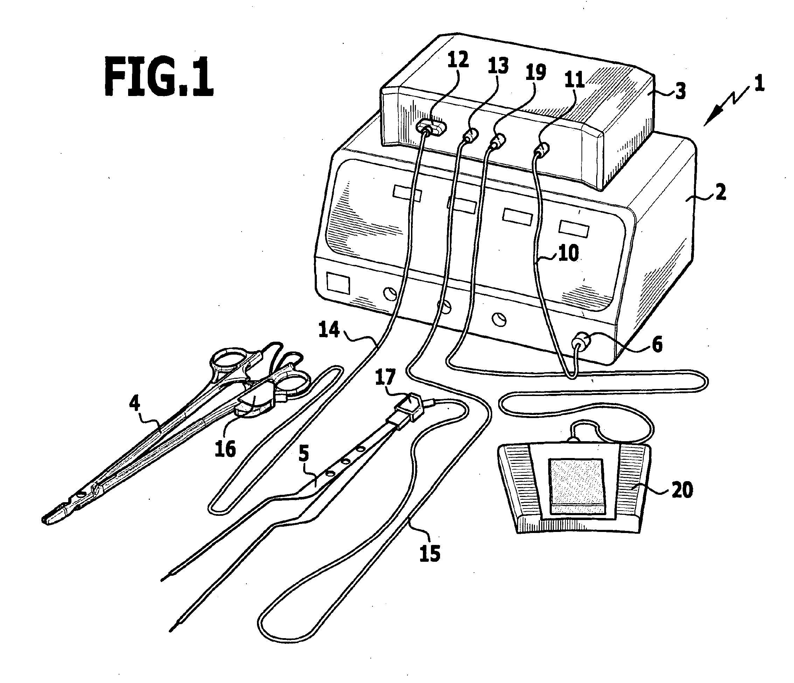

Dieser trägt bei dem Ausführungsbeispiel der Figur 1 zwei Anschlüsse 12, 13, an jeden dieser Anschlüsse 12, 13 ist über ein Anschlusskabel 14 bzw. 15 jeweils eines der beiden HF-Instrumente 4, 5 angeschlossen.

Die beiden HF-Instrumente 4, 5 weisen zusätzlich jeweils einen Handschalter 16 bzw. 17 auf, diese Handschalter 16, 17 sind ebenfalls über die Anschlusskabel 14 und 15 mit den Anschlüssen 12 bzw. 13 verbunden.

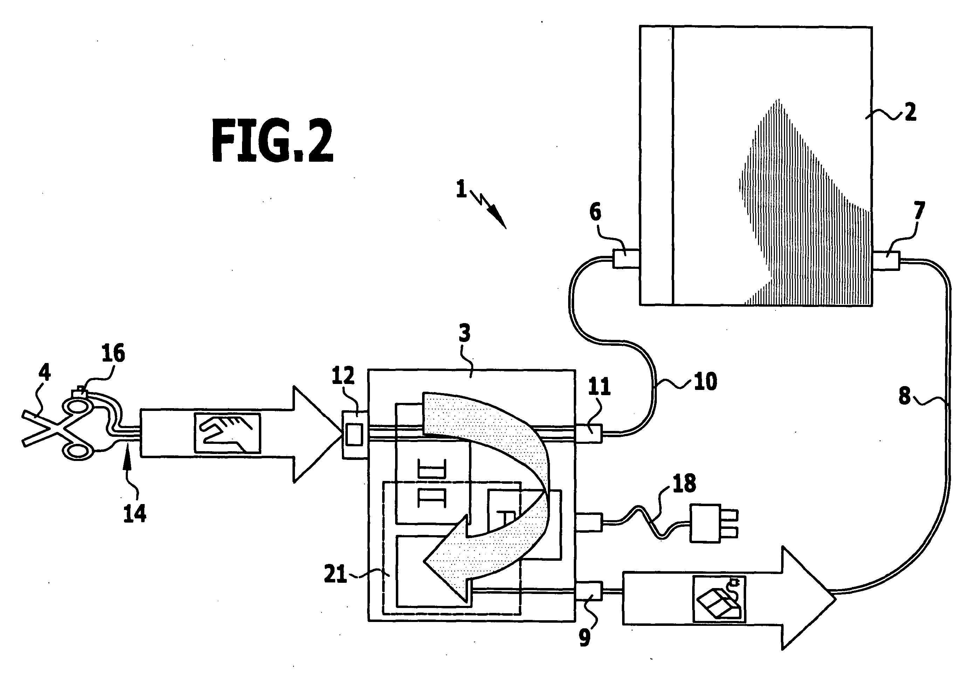

Beim Ausführungsbeispiel der Figur 2 ist eine ähnliche Anordnung gewählt, jedoch ist nur ein Anschluss 12 mit einem daran angeschlossenen HF-Instrument 4 dargestellt.

Der Adapter 3 kann über ein Netzkabel 18 mit elektrischer Energie versorgt werden, und außerdem weist der Adapter 3 beim Ausführungsbeispiel der Figur 1 noch einen Schaltanschluss 19 auf, an den ein Fußschalter 20 angeschlossen werden kann.

Der Adapter 3 ist so ausgebildet, dass er die Anschlüsse 12, 13 mit dem HF-Eingang 11 verbindet und außerdem mit einer elektrischen Schaltung 21, die die Signale der Handschalter 16, 17 verarbeitet und diese mit dem Schaltausgang 9 verbindet. Diese elektrische Schaltung 21 verarbeitet die Signale, die von den Handschaltern 16 oder 17 stammen, so, dass sie am Schaltanschluss 7 des HF-Generators 2 wirksam werden können, so dass die Handschalter funktionsgleich den Fußschaltern arbeiten, die bei einem Betrieb des HF-Generators 2 ohne Adapter Verwendung finden.

Die elektrische Schaltung 21 kann weitere Funktionen erfüllen, beispielsweise kann sie die angeschlossenen HF-Instrumente 4, 5 identifizieren. Diese Identifikation kann in an sich bekannter Weise erfolgen, beispielsweise durch die Erkennung unterschiedlicher Widerstände, die den HF-Instrumenten zugeordnet sind, oder durch Auslesen von Daten aus Transpondern auf den HF-Instrumenten. In jedem Falle werden die entsprechenden Identifikationssignale über die Anschlusskabel 14, 15 auf die Anschlüsse 12, 13 übertragen und von dort der elektrischen Schaltung 21 zugeführt.

Mit der Identifikation der angeschlossenen Instrumente kann die elektrische Schaltung 21 außerdem den jeweiligen HF-Instrumenten entsprechende elektrische Soll-Werte mit den Ist-Werten der elektrischen HF-Energie vergleichen, die vom HF-Generator 2 zugeführt werden, also mit der Einstellung des HF-Generators. Wenn eine einen bestimmten Wert übersteigende Abweichung vorliegt, kann der Adapter ein Warnsignal erzeugen oder die Stromzufuhr abschalten.

Über den Fußschalter 20 kann parallel zu den Handschaltern 16, 17 oder alternativ ebenfalls die elektrische Schaltung 21 mit Schaltsignalen versorgt werden, die dann zur Aktivierung des HF-Generators 2 führen. Bei Verwendung der Handschalter 16, 17 ist also ein solcher Fußschalter 20 nicht unbedingt notwendig, er kann aber vorgesehen werden, falls der Operateur es vorzieht, in herkömmlicher Weise einen Fußschalter zu betätigen. Es ist auch möglich, mit diesem Fußschalter 20 zusätzliche Schaltvorgänge auszulösen, um die Einstellung des HF-Generators 2 zu verändern.

Die Verwendung des Adapters 3 ermöglicht es, HF-Generatoren 2 der konventionellen Bauweise mit einem einzigen HF-Ausgang und einem davon getrennten Schaltanschluss zu verwenden und trotzdem mit Instrumenten zu arbeiten, die Handschalter aufweisen, gegebenenfalls auch mit mehreren Instrumenten, die alternativ eingesetzt werden und durch Betätigung der entsprechenden Handschalter auch als eingeschaltete Instrumente erkannt werden, so dass die HF-Energie nur diesen Instrumenten zugeführt wird, d.h. der Adapter 3 kann aufgrund der Betätigung der Handschalter 16, 17 veranlassen, dass die HF-Energie nur dem jeweiligen Anschluss des Adapters zugeführt wird, an dem eine Betätigung des Handschalters festgestellt worden ist.

Auf diese Weise können konventionelle HF-Generatoren sehr viel vielseitiger eingesetzt werden, ohne dass bauliche Veränderungen notwendig sind, insbesondere können Wechsel beim Anschluss der Instrumente vermieden werden, d.h. alle Instrumente können während einer Operation dauerhaft über den Adapter 3 mit dem HF-Generator verbunden bleiben.

## Patentansprüche

1. HF-Chirurgiegerät (1), umfassend
einen HF-Generator (2), der einen HF-Ausgang (6) und einen Schaltanschluss (7) für ein externes Schaltelement aufweist
über den dem HF-Generator (2) Signale zum Ein- und Ausschalten zugeführt werden können,
und mindestens ein bipolares HF-Instrument (4, 5), dem von dem HF-Ausgang (6) des HF-Generators (2) elektrische HF-Energie zugeführt wird und das einen Handschalter (16, 17) aufweist,
sowie einen Adapter (3) mit mindestens einem Anschluss (12, 13) für das HF-Instrument (4, 5), einem HF-Eingang (11), der mit dem HF-Ausgang des HF-Generators (2) verbunden ist, mit einem Schaltausgang (9) und mit elektrischen Schaltelementen (21), die den Anschluss (12, 13) für das HF-Instrument mit dem HF-Eingang (11) verbinden, **dadurch gekennzeichnet, dass** der Adapter (3) einen mit dem Schaltanschluss (7) am HF-Generator (2) verbundenen Schaltausgang (9) aufweist sowie elektrische Schaltelemente (21), die den Anschluss (12, 13) für das HF-Instrument (4, 5) mit dem Schaltausgang (9) verbinden und die Signale des Handschalters (16, 17) umwandeln in Schaltsignale für den Schaltanschluss (7) des HF-Generators (2), so dass die Handschalter (16, 17) funktionsgleich den externen Schaltelementen arbeiten, die bei einem Betrieb des HF-Generators (2) ohne Adapter Verwendung finden.

2. HF-Chirurgiegerät nach Anspruch 1, **dadurch gekennzeichnet, dass** mehrere Anschlüsse (12, 13) für HF-Instrumente (4, 5) am Adapter (3) vorgesehen sind, die einerseits mit dem HF-Eingang (11) und andererseits mit dem Schaltausgang (9) verbunden sind.

3. HF-Chirurgiegerät nach einem der voranstehenden Ansprüche, **dadurch gekennzeichnet, dass** die HF-Instrumente (4, 5) Identifikationselemente tragen, die über den Anschluss (12, 13) für ein HF-Instrument an eine Erkennungsschaltung (21) im Adapter (3) angeschlossen sind.

4. HF-Chirurgiegerät nach Anspruch 3, **dadurch gekennzeichnet, dass** die Erkennungsschaltung (21) elektrische Sollwerte für das angeschlossene HF-Instrument (4, 5) und elektrische Ist-Werte, die der jeweiligen Einstellung des HF-Generators (2) entsprechen, vergleicht und bei einer Abweichung, die einen vorgegebenen Wert übersteigt, ein Warn- und/oder Abschaltsignal erzeugt.

5. HF-Chirurgiegerät nach einem der voranstehenden Ansprüche, **dadurch gekennzeichnet, dass** der Adapter (3) einen Schaltanschluss (19) für ein externes Schaltelement (20) aufweist, der mit dem Schaltausgang (9) des Adapters (3) in Verbindung steht.

6. HF-Chirurgiegerät nach einem der voranstehenden Ansprüche, **dadurch gekennzeichnet, dass** der Adapter (3) in einem eigenen Gehäuse angeordnet ist.

7. HF-Chirurgiegerät nach Anspruch 6, **dadurch gekennzeichnet, dass** der Adapter (3) über Anschlusskabel (8, 10) mit dem HF-Ausgang (6) und dem Schaltanschluss (7) des HF-Generators (2) verbunden ist.

8. Adapter (3) mit den Merkmalen eines der voranstehenden Ansprüche.

## Claims

1. An HF surgery apparatus (1) comprising an HF generator (2) which has an HF outlet (6) and a switching connection (7) for an external switching element, via which switching connection (7), signals for switching on and switching off can be supplied to the HF generator (2), and comprising at least one bipolar HF instrument (4, 5) to which electrical HF energy is supplied from the HF outlet (6) of the HF generator (2) and which has a manual switch (16, 17), as well as comprising an adaptor (3) with at least one connection (12, 13) for the HF instrument (4,5), with an HF inlet (11) connected to the HF outlet of the HF generator (2), with a switching outlet (9) and with electrical switching elements (21) connecting the connection (12, 13) for the HF instrument to the HF inlet (11), **characterised in that** the adaptor (3) has a switching outlet (9) connected to the switching connection (7) on the HF generator (2) as well as electrical switching elements (21) which connect the connection (12, 13) for the HF instrument (4, 5) to the switching outlet (9) and which convert signals of the manual switch (16, 17) into switching signals for the switching connection (7) of the HF generator (2), so that the manual switches (16, 17) operate in a functionally identical manner to the external switching elements used upon operation of the HF generator (2) without adaptor.

2. An HF surgery apparatus according to claim 1, **characterised in that** a plurality of connections (12, 13) for HF instruments (4, 5) are provided on the adaptor (3) and are connected on the one hand to the HF inlet (11) and on the other hand to the switching outlet (9).

3. An HF surgery apparatus according to any one of the preceding claims, **characterised in that** the HF instruments (4, 5) bear identification elements attached to a recognition circuit (21) in the adaptor (3) via the connection (12, 13) for an HF instrument.

4. An HF surgery apparatus according to claim 3, **characterised in that** the recognition circuit (21) compares electrical desired values for the attached HF instrument (4, 5) and electrical actual values corresponding to the specific adjustment of the HF generator (2), and in the event of a deviation exceeding a predetermined value produces a warning and/or switch-off signal.

5. An HF surgery apparatus according to any one of the preceding claims, **characterised in that** the adaptor (3) has a switching connection (19) for an external switching element (20), which switching connection (19) is connected to the switching outlet (9) of the adaptor (3).

6. An HF surgery apparatus according to any one of the preceding claims, **characterised in that** the adaptor (3) is arranged in its own housing.

7. An HF surgery apparatus according to claim 6, **characterised in that** the adaptor (3) is connected, via connection cables (8, 10), to the HF outlet (6) and to the switching connection (7) of the HF generator (2).

8. An adaptor (3) having the features of one of the preceding claims.

## Revendications

1. Instrument chirurgical HF (1) comprenant
un générateur HF (2), qui présente une sortie HF (6) et un raccord de commutation (7) pour un élément de commutation externe par l'intermédiaire duquel peuvent être amenés au générateur HF (2), des signaux pour la mise en marche ou l'arrêt,
et au moins un instrument HF (4, 5) bipolaire, auquel est amenée de l'énergie électrique HF par la sortie HF (6) du générateur HF (2), et qui comporte un commutateur manuel (16, 17),
ainsi qu'un adaptateur (3) comportant au moins un raccord de branchement (12, 13) pour l'instrument HF (4, 5), une entrée HF (11) reliée à la sortie HF du générateur HF (2), et comportant également une sortie de commutation (9) et des éléments de commutation électriques (21), qui relient le raccord de branchement (12, 13) pour l'instrument HF à l'entrée HF (11),
**caractérisé en ce que** l'adaptateur (3) comporte une sortie de commutation (9) reliée au raccord de commutation (7) sur le générateur HF (2), ainsi que des éléments de commutation électriques (21) qui relient le raccord de branchement (12, 13) pour l'instrument HF (4, 5) à la sortie de commutation (9), et convertissent les signaux du commutateur manuel (16, 17) en signaux de commutation pour le raccord de commutation (7) du générateur HF (2), de sorte que les commutateurs (16, 17) travaillent avec la même fonction que les éléments de commutation externes, qui trouvent leur utilisation lors d'un fonctionnement du générateur HF (2) sans adaptateur.

2. Instrument chirurgical HF selon la revendication 1, **caractérisé en ce que** sur l'adaptateur (3) sont prévus plusieurs raccords de branchement (12, 13) pour des instruments HF (4, 5), raccords qui sont reliés d'une part à l'entrée HF (11) et d'autre part à la sortie de commutation (9).

3. Instrument chirurgical HF selon l'une des revendications précédentes, **caractérisé en ce que** les instruments HF (4, 5) portent des éléments d'identification, qui sont raccordés, par l'intermédiaire du raccord de branchement (12, 13) pour un instrument HF, à un circuit de reconnaissance (21) dans l'adaptateur (3).

4. Instrument chirurgical HF selon la revendication 3, **caractérisé en ce que** le circuit de reconnaissance (21) compare des valeurs de consigne électriques pour l'instrument HF (4, 5) raccordé, et des valeurs électriques réelles, qui correspondent au réglage respectif du générateur HF (2), et, dans le cas d'un écart dépassant une valeur prédéterminée, engendre un signal d'alerte et/ou un signal d'arrêt.

5. Instrument chirurgical HF selon l'une des revendications précédentes, **caractérisé en ce que** l'adaptateur (3) comporte un raccord de commutation (19) pour un élément de commutation externe (20), qui est en liaison avec la sortie de commutation (9) de l'adaptateur (3).

6. Instrument chirurgical HF selon l'une des revendications précédentes, **caractérisé en ce que** l'adaptateur (3) est agencé dans un boîtier qui lui est propre.

7. Instrument chirurgical HF selon la revendication 6, **caractérisé en ce que** l'adaptateur (3) est relié, par l'intermédiaire de câbles de branchement (8, 10), à la sortie HF (6) et au raccord de commutation (7) du générateur HF (2).

8. Adaptateur (3) présentant les caractéristiques de l'une des revendications précédentes.
